# FASCICULE DE BREVET EUROPEEN

(11) **EP 4 619 064 B1**
(45) Date de publication et mention de la délivrance du brevet: **21.10.2026**
(21) Numéro de dépôt: 23822423.2
(22) Date de dépôt: 14.11.2023
(51) Int. Cl.: A61M 15/08, A61M 11/00

(54) **TETE DE DISTRIBUTION NASALE DE PRODUIT FLUIDE**
KOPF ZUR NASALEN ABGABE EINES FLÜSSIGEN PRODUKTS
HEAD FOR NASAL DISPENSING OF A FLUID PRODUCT

(30) Priorité: 14.11.2022 FR 2211798; 16.06.2023 FR 2306234
(43) Date de publication de la demande: 24.09.2025
(73) Titulaire: Aptar France SAS, 27110 Le Neubourg (FR)
(72) Inventeur: BELKHEROUF, Youcef, 27000 EVREUX (FR); DUPRE, Théo, 76000 ROUEN (FR); CAZZOLA, Charles, 76000 ROUEN (FR)
(74) Mandataire: CAPRI
(86) Numéro de dépôt international: PCT/FR2023/051791
(87) Numéro de publication internationale: WO 2024/105336

(56) Documents cités:
- WO-A1-2016/034803
- WO-A1-2021/066195
- WO-A1-2021/069972
- US-A1- 2011 297 145
- US-A1- 2017 360 831
- INTHAVONG K ET AL: "Optimising nasal spray parameters for efficient drug delivery using computational fluid dynamics", COMPUTERS IN BIOLOGY AND MEDICINE, NEW YORK, NY, US, vol. 38, no. 6, 1 June 2008 (2008-06-01), pages 713 - 726, XP022701512, ISSN: 0010-4825, [retrieved on 20080512], DOI: 10.1016/J.COMPBIOMED.2008.03.008

## Description

La présente invention concerne une tête de distribution nasale de produit fluide et un dispositif comportant une telle tête de distribution.

Les dispositifs de distribution nasale sont bien connus. Ils comportent généralement un réservoir contenant une ou plusieurs doses de produits fluide et une tête de distribution mobile par rapport audit réservoir pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, en particulier lorsque le produit fluide distribué a pour objectif d'agir sur le cerveau. En effet, seule une partie minime de la dose atteint généralement la zone cible pour ce type de traitement, à savoir la zone olfactive comprenant les éthmoïdes, notamment en raison de la forme du spray dans la narine qui est conique à la sortie du dispositif. Or, il apparait que cette forme conique n'est pas optimale pour obtenir le maximum de déposition dans la zone cible.

Les documents US1155608 et WO2018109409A1 décrivent des dispositifs d'inhalation nasale qui utilisent un dispositif de positionnement externe pour orienter correctement la tête de distribution dans la narine de l'utilisateur. Cette mise en œuvre améliore la déposition de produit fluide sur la zone cible mais cette amélioration reste limitée en raison de la forme conique du spray à la sortie du dispositif.

Les documents US2017360831, US2011297145, WO2021069972, WO2021066195 et WO2016034803 décrivent d'autres dispositifs de l'état de la technique, mais aucun de ces documents ne permet de générer une distribution de fluide avec deux zones de fluides différentes, avec une seconde zone de fluide élargie par rapport à une première zone de fluide.

La présente invention a pour but de fournir une tête de distribution nasale et un dispositif comportant une telle tête, qui ne reproduisent pas les inconvénients susmentionnés.

La présente invention a notamment pour but de fournir une tête de distribution nasale et un dispositif comportant une telle tête qui améliore le taux de déposition de produit actif sur la zone olfactive et/ou les éthmoïdes.

La présente invention a aussi pour but de fournir une tête de distribution nasale et un dispositif comportant une telle tête permettant de maîtriser la forme du spray dans la narine.

La présente invention a également pour but de fournir une tête de distribution nasale et un dispositif comportant une telle tête qui soient simples et peu coûteux à fabriquer et à assembler.

La présente a donc pour objet une tête de distribution nasale de produit fluide telle que défini dans la revendication 1.

Avantageusement, ladite seconde partie cylindrique proximale prolonge ladite première partie cylindrique distale, et forme à son extrémité axiale ledit orifice de distribution.

Avantageusement, ladite seconde partie cylindrique proximale prolonge ladite première partie cylindrique distale de manière coaxiale le long d'un axe longitudinal de ladite tête de distribution.

Avantageusement, ledit piston est configuré pour coulisser dans ladite première partie cylindrique distale entre une position de repos et une position d'actionnement.

Avantageusement, ledit piston comporte une extension frontale qui en position d'actionnement occupe le volume de ladite seconde partie cylindrique proximale.

Avantageusement, ladite longueur I1 est comprise entre 4mm et 19mm.

Avantageusement, ladite longueur I2 est comprise entre 5mm et 15mm.

Avantageusement, la force d'actionnement F dudit piston est comprise entre 12N et 22N.

Avantageusement, ledit diamètre D1 est notamment environ 2mm.

Avantageusement, ledit diamètre D2 est compris notamment entre 5mm et 20mm.

Avantageusement, le rapport D1/D2 est compris entre 0,1 et 0,4.

Avantageusement, ladite première zone de fluide forme un jet environ axial à travers ledit orifice de sortie, et ladite seconde zone de fluide forme un spray environ conique formant un angle α.

La présente a également pour objet un dispositif de distribution nasale de produit fluide comportant une tête de distribution telle que décrite ci-dessus.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en coupe d'une tête de distribution nasale selon un premier mode de réalisation avantageux, insérée dans une narine,
la figure 2 est une vue schématique en section transversale de la tête de distribution de la figure 1, en position d'actionnement, avec l'illustration des zones de fluides en aval de l'orifice de distribution,
la figure 3 est une vue schématique en section transversale de la tête de distribution de la figure 2, en position d'actionnement, avec l'illustration d'une forme de spray avantageuse en aval de l'orifice de distribution,
la figure 4 est une vue schématique en section transversale de la tête de distribution des figures 2 et 3, en position de repos,
la figure 5 est une vue similaire à celle de la figure 4, en position d'actionnement,
la figure 6 est une vue schématique en section transversale d'une tête de distribution nasale selon un second mode de réalisation avantageux,
la figure 7 est une vue similaire à celle de la figure 6, en position d'actionnement, et
les figures 8 à 10 montrent des distributions de trois fluides de viscosités différentes.

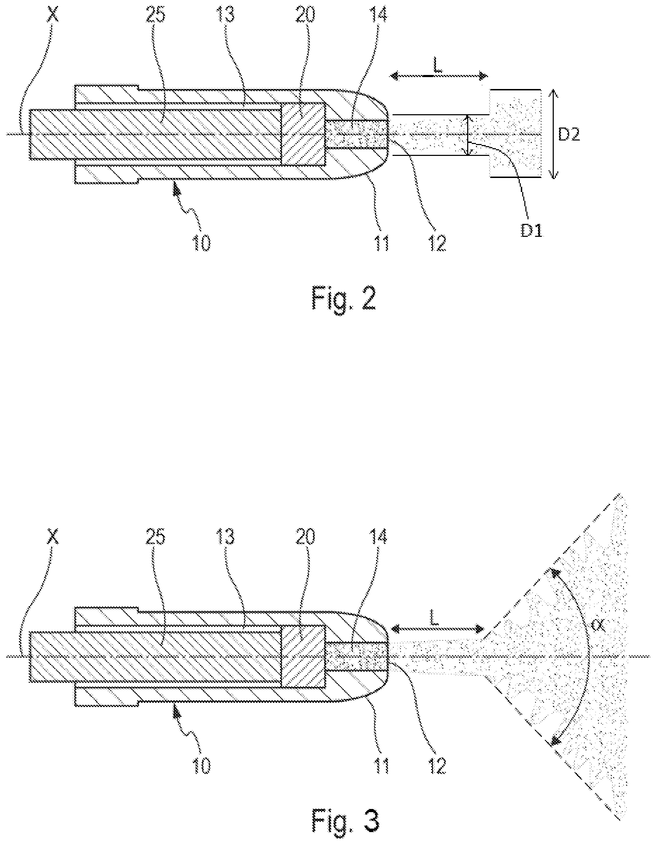

Dans la description, les termes "axial" et "radial" se réfèrent à l'axe longitudinal X du dispositif représenté sur les figures 2 et 3. Les termes "proximal" et "distal" se réfèrent à l'orifice de distribution.

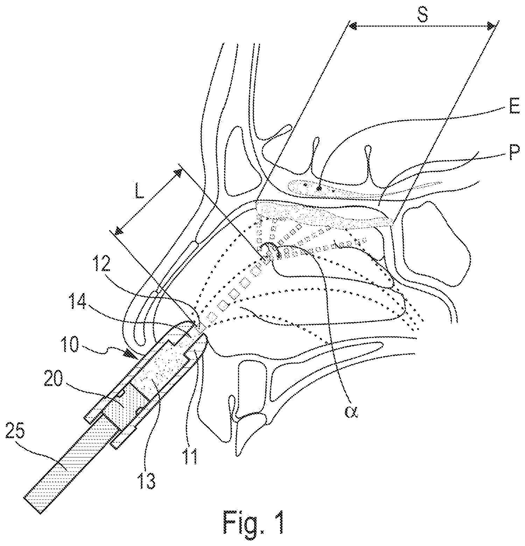

La figure 1 montre une vue en coupe d'une narine. On peut voir le plafond P de la cavité nasale qui comprend les éthmoïdes E. A l'avant de ce plafond P se trouvent la valve nasale et les cornets, dont la présence rend difficile l'accès des éthmoïdes E avec un spray classique, qui s'étend de manière conique à la sortie de l'orifice de distribution 12.

Une tête de distribution 10 comportant un corps 11 pourvu d'un orifice de distribution 12 est insérée dans une narine pour distribuer une dose de produit fluide lors de l'actionnement. La tête de distribution 10 contient un réservoir de produit fluide, avec un piston 20 coulissant axialement dans une partie dudit réservoir pour distribuer le produit fluide à travers l'orifice de distribution.

Le piston 20 est relié à une tige d'actionnement 25 qui peut être actionnée manuellement par l'utilisateur.

En variante, la tête de distribution 10 peut être intégrée dans un dispositif de distribution comportant des moyens de distribution, par exemple un ressort préchargé, adapté à agir sur la tige d'actionnement 25, notamment sur commande de l'utilisateur, par exemple l'actionnement d'un bouton de commande approprié pour déclencher le ressort. Cette solution présente l'avantage de toujours délivrer la même force d'actionnement, de sorte que les propriétés du spray généré lors de l'actionnement sont indépendantes de la manière dont l'utilisateur actionne le dispositif.

Selon l'invention, le produit fluide est distribué d'abord sous la forme d'une première zone de fluide inscrite dans un premier cylindre de diamètre D1 avant de se transformer en une seconde zone de fluide après une distance L à partir de l'orifice de distribution 12, ladite seconde zone de fluide étant inscrite dans un second cylindre de diamètre D2, avec D2 > D1.

Avantageusement, comme visible sur la figure 3, le produit fluide est distribué d'abord sous la forme d'un jet environ axial avant de se transformer en spray environ conique après la distance L à partir de l'orifice de distribution 12.

Comme visible sur la figure 1, l'invention permet au produit fluide de la première zone de fluide de passer au-delà de la valve nasale et des cornets, par exemple en étant sous la forme d'un jet, avant de se transformer en une seconde zone de fluide de plus grand diamètre, par exemple en spray conique, qui va permettre d'atteindre les éthmoïdes E de manière plus importante, à la fois en quantité (part de la dose de fluide qui atteint les éthmoïdes) et en surface (surface S des éthmoïdes touchée par le produit fluide).

Pour réaliser cette forme particulière de double zones de fluide, le réservoir comporte une première partie cylindrique distale 13 de longueur I1 et de diamètre d1 et une seconde partie cylindrique proximale 14 de longueur I2 et de diamètre d2, avec d1 > d2.

La seconde partie cylindrique proximale 14, qui prolonge la première partie cylindrique distale 13, forme à son extrémité axiale l'orifice de distribution 12, qui a donc également un diamètre d2.

De préférence, la seconde partie cylindrique proximale 14 prolonge la première partie cylindrique distale 13 de manière coaxiale le long de l'axe longitudinal X.

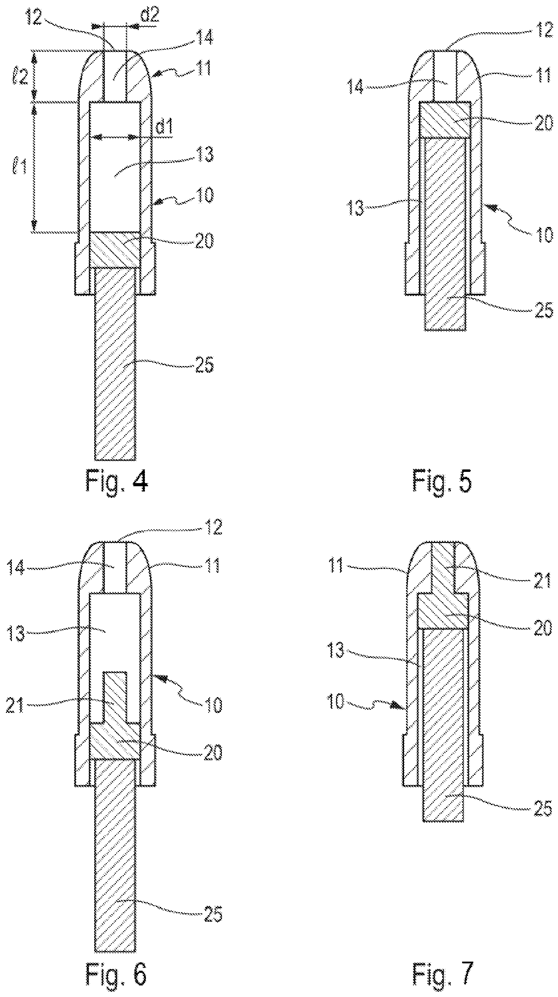

Le piston 20 coulisse dans la première partie cylindrique distale 13 entre une position de repos, visible sur les figures 4 et 6, et une position d'actionnement, visible sur les figures 5 et 7.

Dans le second mode de réalisation des figures 6 et 7, le piston 20 comporte une extension frontale 21 qui en position d'actionnement va occuper le volume de la seconde partie cylindrique proximale 14, pour ainsi réduire le volume mort et distribuer sensiblement la totalité de la dose de produit fluide contenue dans le réservoir 13, 14 avant actionnement.

En se référant aux exemples des figures 2 et 3, en début d'actionnement, un premier volume de produit fluide contenu dans la seconde partie cylindrique proximale 14 est expulsé avec une vitesse V1 à travers l'orifice de distribution 12 pour former une première zone de fluide inscrite dans un cylindre de diamètre D1.

Dans l'exemple de la figure 3, cette première zone de fluide forme un jet environ rectiligne.

En poursuivant la course d'actionnement, un second volume de produit fluide contenu dans la première partie cylindrique distale 13 va passer dans la seconde partie cylindrique proximale 14, avec la réduction du diamètre de d1 à d2 qui provoque une accélération du fluide. Ainsi, le second volume de produit fluide issu de la première partie cylindrique distale 13 va être distribué à travers l'orifice de distribution 12 avec une vitesse V2 supérieure à V1.

Ce second volume de fluide plus rapide va donc rattraper le premier volume de fluide plus lent et le percuter à une distance L de l'orifice de distribution 12, ce qui va générer une explosion et donc former une seconde zone de fluide inscrite dans un cylindre de diamètre D2, avec D2 > D1.

Dans l'exemple de la figure 3, cette seconde zone de fluide forme un spray environ en forme de V, qui définit un angle α.

Ainsi, le spray généré par la présente invention comporte les caractéristiques suivantes importantes pour l'efficacité du dispositif: la longueur L, les diamètres D1 et D2, et éventuellement l'angle α lorsque la seconde zone de fluide forme un spray environ conique.

En faisant varier les caractéristiques de la tête de distribution 10, tel que les longueurs I1 et/ou I2, les diamètres d1 et/ou d2, et/ou celles de l'actionnement du piston, tel que la force d'actionnement du piston 20 et/ou sa vitesse d'actionnement, et/ou le volume de la dose distribuée, on peut faire varier ces paramètres L, D1, D2 et α, et ainsi s'adapter à différentes morphologies, tailles ou formes de narines. En particulier, la longueur L peut être choisie pour générer la seconde zone de spray plus large au bon endroit, qui varie nécessairement selon la personne traitée, adulte/enfant, homme/femme, origines ethniques, etc. De même, la variation du diamètre D2 et/ou de l'angle α permet de moduler la surface S qui sera traitée, qui est aussi variable selon la personne traitée. Une adaptation des paramètres L, D1, D2 et/ou α permet aussi, lorsque souhaitable, de cibler une zone de la cavité nasale autre que les éthmoïdes, par exemple les cornets.

Un autre avantage de la présente invention est qu'il n'est plus nécessaire d'insérer profondément la tête de distribution 10 dans la narine, mais selon la longueur L, un positionnement de l'orifice de distribution 12 à l'entrée de la narine peut être suffisant. Avantageusement dans ce cas, la tête de distribution 10 pourrait comporter des moyens de limitation d'insertion, par exemple une projection radiale disposée autour du corps 11 de la tête de distribution 10, à proximité de l'orifice de sortie 12, et qui pourrait coopérer avec les parois de la narine pour limiter l'insertion.

La tête de distribution comporte selon l'invention les caractéristiques suivantes:
- la longueur I1 est comprise entre 3mm et 20mm, de préférence entre 4mm et 19mm;
- le diamètre d1 est compris entre 2mm et 14mm, de préférence entre 2,9mm et 12,5mm;
- la longueur I2 est comprise entre 3mm et 18mm, de préférence entre 5mm et 15mm;
- le diamètre d2 est compris entre 1mm et 7mm, de préférence entre 2mm et 6mm.

Des tests ont montré qu'un paramètre important pour l'obtention d'un spray tel que souhaité est le rapport des diamètres d2/d1. Selon l'invention, ce rapport d2/d1 est compris entre 0,3 et 0,8, de préférence entre 0,4 et 0,7.

La combinaison des dimensions d1 et d2, et du rapport d2/d1 ci-dessus est requise pour obtenir l'effet souhaité, à savoir la distribution du fluide avec une double zone de fluide, avec une première zone étroite et une seconde zone élargie.

Lorsqu'un ressort préchargé est utilisé pour déplacer le piston et réaliser l'actionnement, le choix de la force du ressort a un impact sur les propriétés du spray généré. La force d'actionnement ou effort F du piston 20 est avantageusement comprise entre 10N et 25N, de préférence entre 12N et 22N. Des tests ont montré qu'avec des caractéristiques de la tête de distribution 10 (I1, d1, I2, d2) qui sont constants, plus la force F est grande, plus l'angle α est petit et plus la longueur L est grande.

Le volume V de la dose de produit fluide distribuée est avantageusement compris entre 0,02ml et 5ml.

Ainsi, la longueur L peut avantageusement varier entre 5mm et 50mm, le diamètre D1 peut avantageusement varier entre 1mm et 3mm, le diamètre D2 peut avantageusement varier entre 3,5mm et 50mm, le rapport D1/D2 peut avantageusement varier entre 0,05 et 0,8, et l'angle α peut avantageusement varier entre 15° et 125°. Avantageusement, le diamètre D1 est d'environ 2mm, le diamètre D2 peut varier entre 5mm et 20mm, et le rapport D1/D2 peut varier entre 0,1 et 0,4.

Le tableau ci-dessous montre différentes configurations testées (avec de l'eau comme produit fluide), pour former des zones de fluides selon l'exemple de la figure 3:

| V (ml) | F (N) | d1 (mm) | I1 (mm) | d2 (mm) | I2 (mm) | d2/d1 | L (mm) | α (°) |
|---|---|---|---|---|---|---|---|---|
| 1 | 12 | 4,69 | 14 | 2 | 5 | 0,43 | 47,21 | 32,05 |
| 3 | 12 | 8,95 | 14 | 4 | 5 | 0,45 | 30,21 | 121,58 |
| 3 | 12 | 8,95 | 14 | 6 | 15 | 0,67 | 15,22 | 31,68 |
| 0,3 | 17 | 2,90 | 9 | 2 | 10 | 0,69 | 34,95 | 18,16 |
| 3 | 17 | 8,95 | 9 | 6 | 15 | 0,67 | 12,81 | 48,49 |
| 3 | 17 | 8,95 | 14 | 6 | 10 | 0,67 | 5,17 | 60,00 |
| 5 | 17 | 12,44 | 4 | 6 | 10 | 0,48 | 20,00 | 25,15 |
| 3 | 22 | 8,95 | 14 | 6 | 5 | 0,67 | **24,79** | 55,34 |

Un réservoir particulièrement adapté à la présente invention comporte les caractéristiques suivantes:
- volume V = 0,2 ml
- longueur I1 = 18,5 (± 0,15) mm;
- diamètre d1 = 3,73 (± 0,01) mm;
- longueur I2 = 11,142 (± 0,11) mm;
- diamètre d2 = 2,025 (± 0,004) mm;
- rapport d2/d1 = 0,54.

Un ressort particulièrement adapté avec ce réservoir est un ressort ayant les caractéristiques suivantes:
- longueur libre = 80 (± 2,5) mm;
- longueur en début d'actionnement = 23,5 mm;
- longueur en fin d'actionnement = 32,8 mm;
- effort F en début d'actionnement (ressort seul) = 19,8 (± 0,5) N;
- effort F en fin d'actionnement (ressort seul) = 16,5 (± 0,5) N;
- raideur (ressort seul) = 0,348 (N/mm);
- effort F en début d'actionnement (ressort assemblé) = 18,9 (± 0,5) N;
- effort F en fin d'actionnement (ressort assemblé) = 12,9 (± 0,5) N;

Ce dispositif particulier a été testé avec trois fluides de viscosités différentes, un premier fluide (eau) avec une viscosité de 2 cP, un second fluide avec une viscosité de 23 cP et un troisième fluide avec une viscosité de 80 cP. Ces tests ont démontré que moins le fluide est visqueux, meilleure est l'obtention d'un spray selon la présente invention.

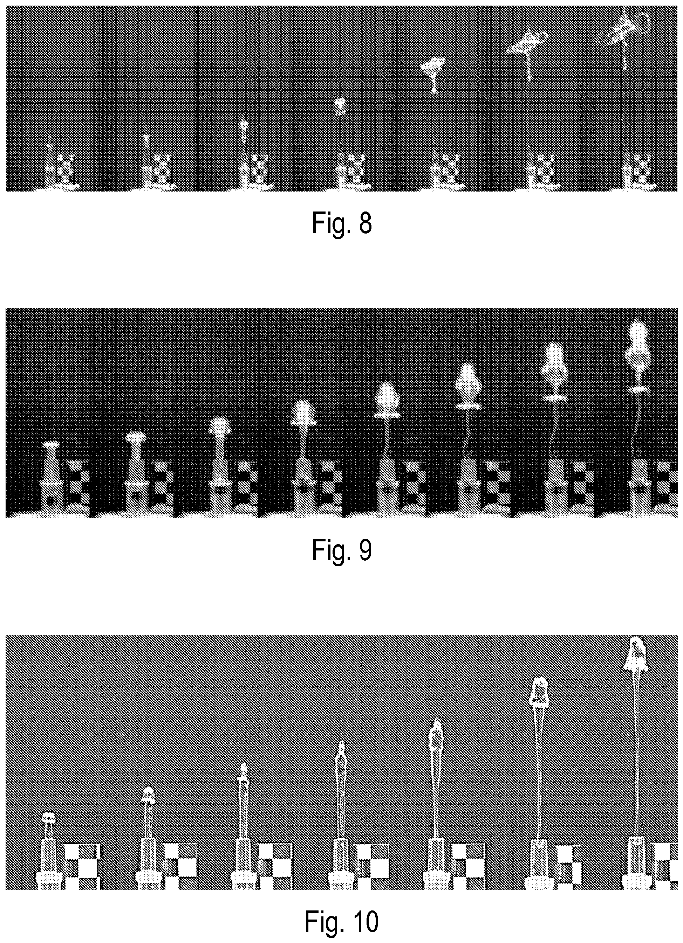

La figure 8 montre un spray obtenu avec le premier fluide, la figure 9 montre un spray obtenu avec le second fluide, et la figure 10 montre un spray obtenu avec le troisième fluide.

Des tests de déposition dans la zone olfactive ont été réalisés avec différentes orientations du dispositif ci-dessus dans une narine artificielle ("nasal cast"), à savoir avec un angle horizontal AH (angle entre l'axe longitudinal du dispositif et un plan horizontal) de 30°, 45° et 60°, et avec un angle vertical AV (angle entre l'axe longitudinal du dispositif et un plan vertical perpendiculaire au visage) de 0°, 5°, 10° et 20°. La profondeur d'insertion PI était de 10 ou 15 mm.

Les tests ci-dessus ont été répétés trois fois, et ont donné les résultats suivants pour les taux de déposition dans la zone olfactive (le tableau contient la moyenne des trois tests):
1. Premier fluide (viscosité 2 cP):

| | AH 30° | AH 45° | AH 60° |
|---|---|---|---|
| AV 0° | | PI = 15 mm: 52% | |
| AV 5° | PI = 15 mm: 37% | PI = 10 mm: 51% | PI = 15 mm: 51% |
| | | PI = 15 mm: 55% | |
| AV 10° | | PI = 15 mm: 54% | |
| AV 20° | | PI = 15 mm: 44% | |

2. Second fluide (viscosité 23 cP):

| | AH 30° | AH 45° | AH 60° |
|---|---|---|---|
| AV 5° | PI = 15 mm: 24% | PI = 10 mm: 50% | PI = 15 mm: 56% |
| | | PI = 15 mm: 57% | |

3. Troisième fluide (viscosité 80 cP):

| | AH 45° |
|---|---|
| AV 5° | PI = 15 mm: 57% |

A titre de comparaison, il a été testé un dispositif standard qui génère un spray conique directement à la sortie du dispositif:
1. Premier fluide (viscosité 2 cP):

| | AH 30° | AH 45° | AH 53° |
|---|---|---|---|
| AV 5° | PI = 15 mm: 5% | PI = 15 mm: 5% | PI = 15 mm: 5% |

2. Second fluide (viscosité 23 cP):

| | AH 45° |
|---|---|
| AV 5° | PI = 15 mm: 9% |

On constate que la présente invention augmente de manière spectaculaire le taux de déposition au niveau de la zone olfactive, passant de moins de 10% pour un dispositif délivrant un spray traditionnel, à plus de 50% dans la majorité des cas avec la présente invention.

Une seringue du marché (Gerresheimer Gx^{®} bulk luer cone syringe 0,5 ml) a été testée à titre comparatif. Cette seringue présente les caractéristiques suivantes:
- volume V = 0,5 ml
- diamètre d1 = 4,65 (± 0,1) mm;
- diamètre d2 = 1,10 (± 0,1) mm;
- rapport d2/d1 = 0,24.

Cette seringue ne permet pas d'obtenir une distribution du fluide avec une double zone de fluide selon la présente invention.

Ceci démontre l'importance des diamètres d1 et d2, et surtout du rapport d2/d1 .

La présente invention est notamment adaptée dans le cas de traitements de maladies neuro dégénératives, type Parkinson ou Alzheimer, nécessitant de faire transiter le médicament au travers de la barrière nez-cerveau. L'atteinte de la zone cible, en particulier les ethmoïdes, par la cavité nasale est un moyen simplifié et non invasif d'administrer un traitement dans le cerveau.

La présente invention peut avantageusement être utilisée avec les médicaments suivants:
- les neurotransmetteurs et les neuromodulateurs, par exemple: acétylcholine (ACH), médicaments anticholinergiques, adénosine triphosphate (ATP), aspartate (Asp), bêta-amyloïde, bêta-endorphine, bradykinine, dopamine (DA), L-DOPA, Carbidopa, épinéphrine, dynorphines, endomorphines, enképhalines, 5-hydroxytryptamine (5-HT), Sumatriptan, Imitrex, Migranal, Zolmitriptan, Zomig, acide gamma-aminobutyrique (GABA), glutamate (glu), glycine, histamine, leptine, facteur de croissance nerveuse et autres facteurs de croissance, noradrénaline, oxyde nitrique, Substance P;
- les anesthésiques généraux, par exemple: alfentanil, desflurane, enflurane, étomidate, fentanyl, halothane, isoflurane, kétamine, méthohexital, méthoxyflurane, midazolam, lorazépam, diazépam, morphine, protoxyde d'azote (N2O), propofol, sévoflurane, Sufentanil, Sublimase, thiopental;
- les anesthésiques locaux, par exemple: benzocaïne, bupivacaïne, cocaïne, lidocaïne, prilocaïne, procaïne, ropivacaïne, tétracaïne;
- les analgésiques opioïdes, les agonistes-antagonistes et les antitussifs, par exemple: agonistes, codéine, diphénoxylate, fentanyl, héroïne et autres opioïdes, hydrocodone, 1-alpha-acétyl-méthadol, acétate de lévométhadyle, lopéramide, mépéridine, méthadone, morphine, oxycodone, d-propoxyphène, combinaisons d'opioïdes plus acétaminophène et asa, tramadol;
- les agonistes/antagonistes et les antagonistes, par exemple: buprénorphine, butorphanol, nalbuphine, nalorphine, naloxone, naltrexone, nalméfène, pentazocine, codéine, dextrométhorphane, hydrocodone; - les médicaments utilisés dans le traitement de la maladie de Parkinson et des troubles moteurs, par exemple: amantadine, apomorphine, baclofène, benzodiazépines, benztropine, bromocriptine, carbidopa, cyclobenzaprine, dantrolène, dopamine, entacapone, halopéridol, L-DOPA, pergolide, pramiprexole, ropinerole, sélégiline (déprényl), trihexyphénidyl, rasagiline, azilect, sélégiline, ladostigil, rotigotine, neupro, inhibiteur de la mono amine oxydase, inhibiteur de la COMT;
- les antiépileptiques, par exemple: acétazolamide, carbamazépine, clonazépam, diazépam, éthosuximide, felbamate, gabapentine, lamotrigine, lorazépam, phénobarbital, phénytoïne, primidone, tiagabine, topiramate, acide valproïque, vigabatrin, midazolam;
- les médicaments utilisés dans les troubles affectifs, par exemple: antidépresseurs, amitriptyline, bupropion, citalopram, clomipramine, désipramine, fluoxétine, fluvoxamine, imipramine, nortriptyline, paroxétine, phénelzine, sertraline, trazodone, tranylcypromine, venlafaxine, médicaments antimaniques, carbamazépine, carbonate de lithium, acide valproïque;
- les antipsychotiques (neuroleptiques), par exemple: chlorpromazine (CPZ), clozapine, fluphénazine, halopéridol, olanzapine, quétiapine, rispéridone, sertindole, thioridazine, thiothixène, ziprasidone;
- les sédatifs-hypnotiques, les anxiolytiques et les myorelaxants à action centrale, par exemple: alprazolam, hydrate de chloral, diphénhydramine, flumazénil, flurazépam, hydroxyzine, lorazépam, oxazépam, phénobarbital, témazépam, triazolam, zaléplon, zolpidem;
- les médicaments utilisés dans les troubles de l'anxiété et les relaxants des muscles squelettiques, par exemple: alprazolam, chlorazépate, chlordiazépoxide, diazépam, flumazénil (antagoniste), lorazépam, oxazépam;
- les traitements de la maladie d'Alzheimer, par exemple: donépézil, galantamine, rivastigmine, tacrine, détémir, novolin, humuline, insuline, hormone analogue à l'insuline, analogue de l'insuline tel que l'insuline NPH, lispro, aspart, insuline détémir, glulisine, insuline glargin, insuline dégludec, BDNF, GDNF, MIBG, agents anticancéreux, médicaments anticancéreux, agonistes de la dopamine, antagonistes de la dopamine;
- d'autres médicaments, par exemple: amphétamine, caféine, éphédrine, méthamphétamine, méthylphénidate, phentermine, sibutramine, disulfirame, éthanol, méthanol, naltrexone, atropine, scopolamine, kétamine, acide lysergique diéthylamide (LSD), MDMA (méthylène dioxy-méthyl amphétamine), mescaline, phencyclidine (PCP), donabinol, marijuana/THC, solvants organiques, nicotine, pentobarbital, composés neuroprotecteurs, peptides neuroprotecteurs, facteurs neuroprotecteurs, davunétide, médicaments anti-schizophréniques, médicaments anti-dépression, comtan, entacopone, agents anti-TDAH, médicaments anti-TDAH tels que le méthylphénidrate (ritaline), médicaments contre l'autisme et les symptômes de l'autisme.

La présente invention a été décrite en référence à des variantes de réalisation avantageuses, mais il est entendu qu'un homme du métier peut y apporter toutes modifications, sans sortir du cadre de la présente invention tel que défini par les revendications annexées.

## Revendications

1. Tête de distribution nasale de produit fluide (10) comportant un corps (11) pourvu d'un orifice de distribution (12), ledit corps (11) formant un réservoir (13, 14) contenant une dose de produit fluide, un piston (20) coulissant dans une partie dudit réservoir pour distribuer ladite dose de produit fluide à travers ledit orifice de distribution (12), **caractérisée en ce que** ladite tête de distribution (10) comporte des moyens pour distribuer ladite dose de produit fluide d'abord sous la forme d'une première zone de fluide inscrite dans un premier cylindre de diamètre D1, puis après une distance L à partir dudit orifice de distribution (12), sous la forme d'une seconde zone de fluide inscrite dans un second cylindre de diamètre D2, avec D2 > D1, ledit diamètre D1 étant compris entre 1mm et 3mm, ledit diamètre D2 étant compris entre 3,5mm et 50mm, et le rapport D1/D2 est compris entre 0,05 et 0,8, ledit réservoir comportant une première partie cylindrique distale (13) de longueur I1 et de diamètre d1 et une seconde partie cylindrique proximale (14) de longueur I2 et de diamètre d2, avec d1 > d2, ladite longueur I1 étant comprise entre 3mm et 20mm, ladite longueur I2 étant comprise entre 3mm et 18mm, ledit diamètre d1 étant compris entre 2mm et 14mm, ledit diamètre d2 étant compris entre 1mm et 7mm, et le rapport d2/d1 étant compris entre 0,3 et 0,8, la force d'actionnement F dudit piston (20) étant comprise entre 10N et 25N, et le volume V de la dose de produit fluide distribuée étant compris entre 0,02ml et 5ml.

2. Tête selon la revendication 1, dans laquelle ladite seconde partie cylindrique proximale (14) prolonge ladite première partie cylindrique distale (13), et forme à son extrémité axiale ledit orifice de distribution (12).

3. Tête selon la revendication 2, dans laquelle ladite seconde partie cylindrique proximale (14) prolonge ladite première partie cylindrique distale (13) de manière coaxiale le long d'un axe longitudinal (X) de ladite tête de distribution (10).

4. Tête selon l'une quelconque des revendications précédentes, dans laquelle ledit piston (20) est configuré pour coulisser dans ladite première partie cylindrique distale (13) entre une position de repos et une position d'actionnement.

5. Tête selon l'une quelconque des revendications précédentes, dans laquelle ledit piston (20) comporte une extension frontale (21) qui en position d'actionnement occupe le volume de ladite seconde partie cylindrique proximale (14).

6. Tête selon l'une quelconque des revendications précédentes, dans laquelle ladite longueur I1 est comprise entre 4mm et 19mm.

7. Tête selon l'une quelconque des revendications précédentes, dans laquelle ladite longueur I2 est comprise entre 5mm et 15mm.

8. Tête selon l'une quelconque des revendications précédentes, dans laquelle ledit diamètre d1 est compris entre 2,9mm et 12,5mm.

9. Tête selon l'une quelconque des revendications précédentes, dans laquelle ledit diamètre d2 est compris entre 2mm et 6mm.

10. Tête selon l'une quelconque des revendications précédentes, dans laquelle le rapport d2/d1 est compris entre 0,4 et 0,7.

11. Tête selon l'une quelconque des revendications précédentes, dans laquelle ledit diamètre D1 est environ 2mm.

12. Tête selon l'une quelconque des revendications précédentes, dans laquelle ledit diamètre D2 est compris entre 5mm et 20mm.

13. Tête selon l'une quelconque des revendications précédentes, dans laquelle le rapport D1/D2 est compris entre 0,1 et 0,4.

14. Tête selon l'une quelconque des revendications précédentes, dans laquelle ladite première zone de fluide forme un jet environ axial à travers ledit orifice de sortie (12), et ladite seconde zone de fluide forme un spray environ conique formant un angle α.

15. Dispositif de distribution nasale de produit fluide, **caractérisé en ce qu'**il comporte une tête de distribution selon l'une quelconque des revendications précédentes.

## Patentansprüche

1. Kopf zur nasalen Abgabe eines flüssigen Produkts (10), umfassend einen Körper (11), der mit einer Abgabeöffnung (12) versehen ist, wobei der Körper (11) einen Behälter (13, 14) bildet, der eine Dosis des flüssigen Produkts enthält, einen Kolben (20), der in einem Teil des Behälters gleitet, um die Dosis des flüssigen Produkts durch die Abgabeöffnung (12) hindurch abzugeben,
**dadurch gekennzeichnet, dass** der Abgabekopf (10) Mittel zum Abgeben der Dosis des flüssigen Produkts umfasst, zunächst in Form eines ersten Flüssigkeitsbereichs, der in einem ersten Zylinder mit einem Durchmesser D1 eingeschrieben ist, dann nach einem Abstand L von der Abgabeöffnung (12) aus in Form eines zweiten Flüssigkeitsbereichs, der in einem zweiten Zylinder mit einem Durchmesser D2 eingeschrieben ist, wobei D2 > D1, wobei der Durchmesser D1 zwischen 1 mm und 3 mm liegt, wobei der Durchmesser D2 zwischen 3,5 mm und 50 mm liegt, und das Verhältnis D1/D2 zwischen 0,05 und 0,8 liegt, wobei der Behälter einen ersten distalen zylindrischen Teil (13) mit einer Länge l1 und einem Durchmesser d1 und einen zweiten proximalen zylindrischen Teil (14) mit einer Länge l2 und einem Durchmesser d2 umfasst, wobei d1 > d2, wobei die Länge l1 zwischen 3 mm und 20 mm liegt, die Länge l2 zwischen 3 mm und 18 mm liegt, der Durchmesser d1 zwischen 2 mm und 14 mm liegt, der Durchmesser d2 zwischen 1 mm und 7 mm liegt, und das Verhältnis d2/d1 zwischen 0,3 und 0,8 liegt, wobei die Betätigungskraft F des Kolbens (20) zwischen 10 N und 25 N liegt, und das Volumen V der Dosis des abgegebenen flüssigen Produkts zwischen 0,02 ml und 5 ml liegt.

2. Kopf nach Anspruch 1, wobei der zweite proximale zylindrische Teil (14) den ersten distalen zylindrischen Teil (13) verlängert und an seinem axialen Ende die Ausgabeöffnung (12) bildet.

3. Kopf nach Anspruch 2, wobei der zweite proximale zylindrische Teil (14) den ersten distalen zylindrischen Teil (13) entlang einer Längsachse (X) des Abgabekopfs (10) koaxial verlängert.

4. Kopf nach einem der vorhergehenden Ansprüche, wobei der Kolben (20) dazu konfiguriert ist, in dem ersten distalen zylindrischen Teil (13) zwischen einer Ruheposition und einer Betätigungsposition zu gleiten.

5. Kopf nach einem der vorhergehenden Ansprüche, wobei der Kolben (20) eine frontale Erweiterung (21) umfasst, die in der Betätigungsposition das Volumen des zweiten proximalen zylindrischen Teils (14) einnimmt.

6. Kopf nach einem der vorhergehenden Ansprüche, wobei die Länge l1 zwischen 4 mm und 19 mm liegt.

7. Kopf nach einem der vorhergehenden Ansprüche, wobei die Länge l2 zwischen 5 mm und 15 mm liegt.

8. Kopf nach einem der vorhergehenden Ansprüche, wobei der Durchmesser d1 zwischen 2,9 mm und 12,5 mm liegt.

9. Kopf nach einem der vorhergehenden Ansprüche, wobei der Durchmesser d2 zwischen 2 mm und 6 mm liegt.

10. Kopf nach einem der vorhergehenden Ansprüche, wobei das Verhältnis d2/d1 zwischen 0,4 und 0,7 liegt.

11. Kopf nach einem der vorhergehenden Ansprüche, wobei der Durchmesser D1 ungefähr 2 mm beträgt.

12. Kopf nach einem der vorhergehenden Ansprüche, wobei der Durchmesser D2 zwischen 5 mm und 20 mm liegt.

13. Kopf nach einem der vorhergehenden Ansprüche, wobei das Verhältnis D1/D2 zwischen 0,1 und 0,4 liegt.

14. Kopf nach einem der vorhergehenden Ansprüche, wobei der erste Flüssigkeitsbereich einen ungefähr axialen Strahl durch die Ausgabeöffnung (12) hindurch bildet, und der zweite Flüssigkeitsbereich ein ungefähr kegelförmiges Spray bildet, das einen Winkel α bildet.

15. Vorrichtung zur nasalen Abgabe eines flüssigen Produkts, **dadurch gekennzeichnet, dass** sie einen Abgabekopf nach einem der vorhergehenden Ansprüche umfasst.

## Claims

1. A nasal dispensing head of fluid product (10) comprising a body (11) provided with a dispensing orifice (12), said body (11) forming a reservoir (13, 14) containing a dose of fluid product, a piston (20) sliding in a portion of said reservoir to dispense said dose of fluid product through said dispensing orifice (12), **characterised in that** said dispensing head (10) comprises means to dispense said dose of fluid product, first in the form of a first fluid zone inscribed in a first cylinder of diameter D1, then after a distance L from said dispensing orifice (12), in the form of a second fluid zone inscribed in a second cylinder of diameter D2, with D2 > D1, said diameter D1 being of between 1 mm and 3 mm, said diameter D2 being of between 3.5 mm and 50 mm, and the ratio D1/D2 is of between 0.05 and 0.8, said reservoir comprising a first distal cylindrical portion (13) of length l1 and of diameter d1 and a second proximal cylindrical portion (14) of length l2 and of diameter d2, with d1 > d2, said length l1 being of between 3 mm and 20 mm, said length l2 being of between 3 mm and 18 mm, said diameter d1 being of between 2 mm and 14 mm, said diameter d2 being of between 1 mm and 7 mm, and the ratio d2/d1 being of between 0.3 and 0.8, the actuation force F of said piston (20) being of between 10N and 25N, and the volume V of the dose of fluid product dispensed being of between 0.02 ml and 5 ml.

2. The head according to claim 1, wherein said second proximal cylindrical portion (14) extends said first distal cylindrical portion (13), and forms at its axial end said dispensing orifice (12).

3. The head according to claim 2, wherein said second proximal cylindrical portion (14) extends said first distal cylindrical portion (13) coaxially along a longitudinal axis (X) of said dispensing head (10).

4. The head according to any one of the preceding claims, wherein said piston (20) is configured to slide in said first distal cylindrical portion (13) between a rest position and an actuation position.

5. The head according to any one of the preceding claims, wherein said piston (20) comprises a front extension (21) which in the actuation position occupies the volume of said second proximal cylindrical portion (14).

6. The head according to any one of the preceding claims, wherein said length l1 is of between 4 mm and 19 mm.

7. The head according to any one of the preceding claims, wherein said length l2 is of between 5 mm and 15 mm.

8. The head according to any one of the preceding claims, wherein said diameter d1 is of between 2.9 mm and 12.5 mm.

9. The head according to any one of the preceding claims, wherein said diameter d2 is of between 2 mm and 6 mm.

10. The head according to any one of the preceding claims, wherein the ratio d2/d1 is of between 0.4 and 0.7.

11. The head according to any one of the preceding claims, wherein said diameter D1 is about 2 mm.

12. The head according to any one of the preceding claims, wherein said diameter D2 is of between 5 mm and 20 mm.

13. The head according to any one of the preceding claims, wherein the ratio D1/D2 is of between 0.1 and 0.4.

14. The head according to any one of the preceding claims, wherein said first fluid zone forms an approximately axial jet through said outlet orifice (12), and said second fluid zone forms an approximately conical spray forming an angle α.

15. A device for nasal dispensing of fluid product, **characterised in that** it comprises a dispensing head according to any one of the preceding claims.
